**Europäisches Patentamt**

**European Patent Office**

**Office européen des brevets**

(19)

(11) Veröffentlichungsnummer : **0 065 727
B1**

# (12) EUROPÄISCHE PATENTSCHRIFT

(45) Veröffentlichungstag der Patentschrift :
**25.07.84**

(21) Anmeldenummer : **82104266.0**

(22) Anmeldetag : **15.05.82**

(51) Int. Cl.³ : **C 07 C118/02// B01J19/26**

(54) **Verfahren zur kontinuierlichen Herstellung von organischen Mono- oder Polyisocyanaten.**

(30) Priorität : **27.05.81 DE 3121036**

(43) Veröffentlichungstag der Anmeldung :
**01.12.82 Patentblatt 82/48**

(45) Bekanntmachung des Hinweises auf die Patenterteilung : **25.07.84 Patentblatt 84/30**

(84) Benannte Vertragsstaaten :
**BE DE FR GB IT NL**

(56) Entgegenhaltungen :
**DE-C- 1 146 872
FR-A- 2 325 637**

(73) Patentinhaber : **BAYER AG
Konzernverwaltung RP Patentabteilung
D-5090 Leverkusen 1 Bayerwerk (DE)**

(72) Erfinder : **Hennig, Hans-Joachim, Dr.
Saarbrückerstrasse 42
D-5090 Leverkusen 1 (DE)**
Erfinder : **Lahrs, Jürgen, Dr.
Volberger Weg 11
D-5000 Köln 91 (DE)**
Erfinder : **Liebsch, Dieterich, Dr.
Nietzschestrasse 11
D-5090 Leverkusen (DE)**

EP 0 065 727 B1

## Beschreibung

Die Erfindung betrifft ein verbessertes Verfahren zur kontinuierlichen Herstellung von organischen Mono- oder Polyisocyanaten durch kontinuierliche Phosgenierung der den Isocyanaten zugrundeliegenden primären Amine.

Es ist bereits bekannt, organische Isocyanate in kontinuierlicher Verfahrensweise durch Umsetzung primärer Amine mit Phosgen bei erhöhten Drücken und Temperaturen herzustellen.

So beschreibt die DE-OS 2 252 068 die Herstellung von Isocyanaten bei überatmosphärischen Drücken, wobei das gewünschte Isocyanat als Lösungsmittel für das benötigte Phosgen dient.

Unter ähnlichen Druck- und Temperaturbedingungen wird gemäß DE-AS 1 768 439 eine Aminlösung mit reinem Phosgen zur Reaktion gebracht.

Schließlich beschreibt die DE-OS 2 404 773 die Umsetzung von primären Aminen mit Phosgen unter Druck unter Ausschluß von Lösungsmitteln, wobei Phosgen als letzeres dient.

Da es sich bei der Umsetzung von primärem Amin mit Phosgen um eine äußerst schnell ablaufende Reaktion handelt, wirkt sich optimales Vermischen direkt auf Reaktionszeit und besonders auf die Isocyanat-Ausbeute aus. Mehrere Veröffentlichungen befassen sich daher auch mit der Vereinigung der beiden Reaktionskomponenten. So beschreibt beispielsweise US-PS 3 781 320 einen speziellen Ringspalt-Mischer und FR-PS 2 325 637 eine Mischvorrichtung, die den Strom zumindest einer Komponente in eine kreisende und gegenläufige Bewegung versetzt. Auch ein Verfahren zur Vermischung von zwei Flüssigkeiten, nämlich Amin- und Phosgenlösung, bei denen fächerförmige Strahlen ausgebildet werden, wurde bekannt (DE-OS 2 950 216). Die DE-OS 1 792 660 beansprucht schließlich eine Vorrichtung zum Vermischen zweier Ströme flüssiger Reaktionsteilnehmer, die es gestattet, die Ströme unter einem spitzen Winkel zusammenzuführen.

Das nachstehend näher beschriebene erfindungsgemäße Verfahren stellt insofern eine ganz wesentliche Bereicherung des Standes der Technik dar, als es erstmals die Möglichkeit eröffnet, organische Mono- oder Polyisocyanate unter gleichzeitiger Gewährleistung folgender Vorteile herzustellen :

1. Die Phosgenierungsreaktion kann kontinuierlich und einstufig durchgeführt werden.

2. Der zur Durchführung des Verfahrens erforderliche apparative Aufwand ist äußerst gering.

3. Das Verfahren gestattet die Herstellung von organischen Mono- oder Polyisocyanaten in bislang nicht bekannt gewordenen Raum/Zeit-Ausbeuten bei gleichzeitig ausgezeichneten Produktausbeuten.

4. Störende Nebenreaktionen, insbesondere die Bildung von die Vorrichtung und Rohrleitungen verstopfenden Nebenprodukten werden nicht beobachtet.

Gegenstand der Erfindung ist ein Verfahren zur kontinuierlichen Herstellung von organischen Mono- oder Polyisocyanaten in einstufiger Reaktion durch kontinuierliche Vereinigung von Lösungen primärer Mono- oder Polyamine in inerten organischen Lösungsmitteln mit überschüssigen Mengen an, in einem inerten organischen Lösungsmittel gelöstem, Phosgen bei erhöhtem Druck und erhöhter Temperatur in einem Mischraum (4) und kontinuierliche Aufarbeitung der den Mischraum kontinuierlich verlassenden vereinigten Lösungen, gegebenenfalls nachdem diese eine dem Mischraum nachgeschaltete Reaktionszone durchlaufen haben, dadurch gekennzeichnet, daß man die im Überschuß zum Einsatz gelangende Phosgenkomponente kontinuierlich in dem Mischraum vorlegt und die im Unterschuß zum Einsatz gelangende Aminkomponente mittels mindestens einer Glattstrahldüse (2) mit einem lichten Durchmesser von 0,1 bis 30 mm unter Einhaltung eines Differenzdrucks von mindestens 0,5 bar in die vorgelegte Phosgenlösung eindüst.

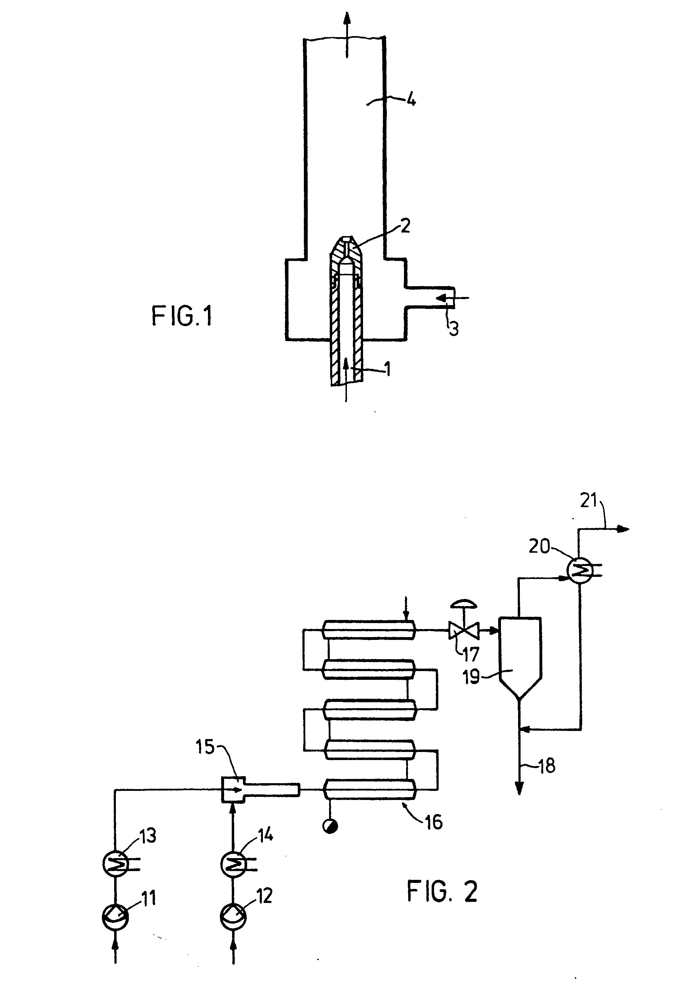

Das erfindungsgemäße Verfahren bzw. die zu seiner Durchführung erforderliche Mischvorrichtung seien zunächst anhand der Figuren 1 und 2 erläutert :

Figur 1 zeigt den mit der erfindungswesentlichen Glattstrahldüse versehenen Mischraum. Im einzelnen bedeuten

(1) die in den Mischraum führende Rohrleitung für die Aminlösung ;
(2) die Glattstrahldüse ;
(3) die zum Mischraum führende Rohrleitung für die Phosgenlösung und
(4) den Mischraum.

Figur 2 zeigt ein für die Durchführung des erfindungsgemäßen Verfahrens beispielsweise geeignetes Fließschema. Im einzelnen bedeuten

(11) eine Pumpe für die Aminlösung ;
(12) eine Pumpe für die Phosgenlösung ;
(13) einen zur Erhitzung der Aminlösung geeigneten Wärmeaustauscher ;
(14) einen zur Erhitzung der Phosgenlösung geeigneten Wärmeaustauscher ;
(15) einen mit einer Glattstrahldüse versehenen Mischraum gemäß Fig. 1 ;
(16) einen dem Mischraum nachgeschalteten, beheizbaren Röhrenreaktor ;
(17) ein Entspannungsventil ;
(18) die Entnahmestelle für Isocyanatlösung ;
(19) ein Entspannungsgefäß ;
(20) einen Kühler und
(21) die Entnahmestelle für die anfallende Gasphase.

Das erfindungsgemäße Verfahren eignet sich zur Phosgenierung beliebiger primärer Mono- und Polyamine, insbesondere zur Herstellung der in der Polyurethanchemie üblichen Polyisocyanate. Ausgangsmaterialien für das erfindungsgemäße Verfahren sind somit neben 3 bis 95 gew.-%igen, vorzugsweise 20 bis 75 gew.-%igen Phosgenlösungen in geeigneten Lösungsmitteln, 5 bis 95 gew.-%ige, vorzugsweise 5 bis 50 gew.-%ige Lösungen von Mono- oder Polyaminen in geeigneten Lösungsmitteln. Für das erfindungsgemäße Verfahren geeignete Amine sind beliebige primäre Mono- oder Polyamine wie z. B. Methylamin, Ethylamin, Butylamin, Stearylamin, Phenylamin, p-Tolylamin, 1,4-Diaminobutan, 1,6-Diaminohexan, 1,8-Diaminooctan, 1,4-Diaminobenzol, 2,4-Diaminotoluol, 2,6-Diaminotoluol, Gemische der beiden letztgenannten Isomeren, 2,2'-Diaminodiphenylmethan, 2,4'-Diaminodiphenylmethan, 4,4'-Diaminodiphenylmethan, Gemische der drei letztgenannten Isomeren, Alkyl-substituierte Diamine der Diphenylmethanreihe wie z. B. 3,4'-Diamino-4-methyldiphenylmethan, Polyamingemische der Diphenylmethanreihe, wie sie in an sich bekannter Weise durch Anilin/Formaldehyd-Kondensation erhalten werden, p-Xylylendiamin, perhydriertes 2,4- und/oder 2,6-Diaminotoluol, 2,2'-, 2,4'- und/oder 4,4'-Diaminodicyclohexylmethan, 1-Amino-3,3,5-trimethyl-5-aminomethyl-cyclohexan (Isophorondiamin, abgekürzt IPDA), Lysin-ethylester, Lysinaminoethylester oder 1,6,11-Triamino-undecan.

Geeignete Lösungsmittel zur Herstellung der Phosgen- und Aminlösungen sind beliebige, unter den Reaktionsbedingungen inerte Lösungsmittel wie z. B. Chlorbenzol, o-Dichlorbenzol, Toluol, Xylol, Methylenchlorid, Perchlorethylen, Trichlorfluormethan oder Butylacetat. Vorzugsweise werden Chlorbenzol oder o-Dichlorbenzol eingesetzt. Beliebige Gemische der beispielhaft genannten Lösungsmittel können selbstverständlich auch eingesetzt werden. Zweckmäßigerweise wird man für die Aminkomponente und das Phosgen das gleiche Lösungsmittel bzw. Lösungsmittelgemisch einsetzen, obwohl dies nicht unbedingt erforderlich ist.

Bei der Durchführung des erfindungsgemäßen Verfahrens kommen die Phosgen- und Aminlösungen in solchen Mengen zum Einsatz, daß im Mischraum ein Molverhältnis Phosgen : primäre Aminogruppen von ca. 2 : 1 bis 30 : 1, vorzugsweise 3 : 1 bis 18 : 1, vorliegt.

Erfindungswesentlich ist die Art der Vereinigung der Reaktionspartner. Hierzu bedient man sich erfindungsgemäß eines mit einer Glattstrahldüse versehenen Mischraums, wie er beispielhaft durch die Fig. 1 dargestellt wird. Die Glattstrahldüse weist einen lichten Durchmesser von 0,1 bis 30 mm, vorzugsweise von 0,5 bis 15 mm und eine Länge von mindestens 0,1 mm, vorzugsweise von 1 bis 15 mm, auf, wobei das Verhältnis von Länge zu Durchmesser mindestens 1 : 1 betragen sollte. Der Innendurchmesser des Mischraums sollte zweckmäßigerweise mindestens das 10-fache, vorzugsweise das 50- bis 300-fache des lichten Durchmessers der Glattstrahldüse betragen, so daß der Abstand des Düsenstrahls zur Seitenwand des Mischraums mindestens das 5-fache des lichten Düsendurchmessers ausmacht. Der Abstand vom Düsenaustritt und der gegenüberliegenden Begrenzung des Mischraums bzw. der dem Mischraum nachgeschalteten Reaktionszone sollte mindestens das 10-fache, vorzugsweise mindestens 100-fache des Düsendurchmessers betragen um sicherzustellen, daß der Düsenstrahl unbehindert von, der Düse gegenüberliegenden, Begrenzungen der Mischzone in die Phosgenlösung eindringen kann.

Das erfindungsgemäße Verfahren kann im Prinzip auch unter Verwendung eines Mischraums durchgeführt werden, der anstelle einer einzigen Glattstrahldüse mehrere parallel angeordnete Glattstrahldüsen der genannten Art aufweist. In einem solchen Falle werden die Glattstrahldüsen so angeordnet, daß die die Düse verlassenden Strahlen der Aminlösung parallel in die vorgelegte Phosgenlösung eindringen, und daß der Abstand zwischen den einzelnen Strahlen mindestens das 10-fache und der Abstand zwischen dem bzw. den äußeren Düsenstrahl(en) zur Seitenwand des Mischraums mindestens das 10-fache des Düsendurchmessers betragen. Es ist prinzipiell auch möglich, parallel angeordnete Düsen zu verwenden, die einen unterschiedlichen lichten Durchmesser aufweisen. Vorzugsweise wird das erfindungsgemäße Verfahren jedoch mit einer Mischvorrichtung durchgeführt, die lediglich eine Glattstrahldüse aufweist.

Bei der Durchführung des erfindungsgemäßen Verfahrens wird das Mischgefäß und die gegebenenfalls dem Mischgefäß nachgeschaltete Reaktionszone kontinuierlich mit der auf 120 bis 300 °C, vorzugsweise 150 bis 250 °C erhitzten Phosgenlösung beschickt, wobei das Auslaßventil im allgemeinen so eingestellt wird, daß in dem Mischgefäß und der gegebenenfalls nachgeschalteten Reaktionszone ein Druck von 10 bis 1 000, vorzugsweise 25 bis 150 bar, besteht, so daß die Phosgenlösung das Mischgefäß einphasig, d. h. ohne Bildung von Gasblasen, kontinuierlich durchströmt und vollständig ausfüllt. Man kann jedoch das Auslaßventil auch so einstellen, daß bei der gewählten Temperatur das Reaktionsgemisch, d. h. insbesondere der sich bildende Chlorwasserstoff teilweise gasförmig vorliegt, so daß insbesondere in der dem Mischraum nachgeschalteten Reaktionszone ein zweiphasiges System vorliegt. Das Durchströmen der bevorzugt als nachgeschaltete Reaktionszone verwendeten Röhrenreaktoren mit zweiphasigen Gemischen bewirkt ein zusätzliches intensives Durchmischen der Reaktionspartner. Selbstverständlich muß der Druck jedoch so eingestellt sein, daß die die Düse verlassende Aminlösung in eine flüssige Phosgenlösung eindringt. In die den Mischraum und die gegebenenfalls nachgeschaltete Reaktionszone unter den genannten Druck- und Temperaturbedingungen kontinuierlich durchfließende Phosgenlösung wird dann bei der Durchführung des erfindungsgemäßen Verfahrens die Aminlösung mittels der bzw. den Glattstrahldüse(n) unter Aufrechterhaltung eines Differenzdrucks von mindestens 0,5 bar, vorzugsweise von 1 bis 200 bar, insbesondere von 3 bis 50 bar, eingedüst, d. h. der durch die Pumpe für die Aminlösung aufgebaute Druck muß vor der bzw. den Glattstrahldüse(n) entsprechend höher als der im Mischraum, durch die Pumpe für die Phosgenlösung aufgebaute Druck, sein.

Das Eintragen der Aminlösung in Form eines äußerst feinen Strahles in die Phosgenlösung mit hoher Geschwindigkeit, wie es eine Glattstrahldüse ermöglicht, gewährt eine intensive Vermischung der Komponenten und deren gleichzeitig ablaufende Reaktion. Die Mischqualität, die sich auf die gleichzeitig ablaufende Reaktion und folglich auf die Isocyanat-Ausbeute auswirkt, wird entscheidend von der Höhe des Differenzdruckes, mit dem man die Aminlösung in die unter Druck stehende Phosgenlösung eindüst, beeinflußt, da dieser die Geschwindigkeit, mit der der Amin-Feinstrahl in die Phosgenlösung eindringt, bestimmt.

Zweckmäßigerweise sollte der Druck der Aminlösung im Vergleich zu dem der Phosgenlösung, d. h. der Differenzdruck der beiden Komponenten, innerhalb der genannten Bereiche so gewählt werden, daß die eingedüste Aminlösung mit einer relativen Geschwindigkeit von mehr als 5 m/sek in die vorgelegte Phosgenlösung eindringt. Bei miteinander äußerst schnell reagierenden Systemen, wie es die Umsetzung von primärem Amin und Phosgen darstellt, ist höchste Mischqualität erforderlich, die durch ein hohes Schergefälle erzielt werden muß. Deshalb sollte das Geschwindigkeitsverhältnis der sich im Gleichstrom, jedoch mit unterschiedlichen Geschwindigkeiten bewegenden und sich folglich scherenden Flüssigkeiten mindestens 5 : 1 betragen. Diese Bedingungen sind mit Differenzdrücken innerhalb der genannten Bereiche zu erzielen.

Die Temperatur der einzudüsenden Aminlösung ist nicht kritisch, da die Temperatur im Mischgefäß und der gegebenenfalls nachgeschalteten Reaktionszone in erster Linie durch die Temperatur der im Überschuß vorliegenden Phosgenlösung, die Reaktionswärme der exothermen Phosgenierungsreaktion und die äußere Heizung der gegebenenfalls der Mischkammer nachgeschalteten Reaktionszone vorgegeben ist. Im allgemeinen wird die einzudüsende Aminlösung auf ca. 20 bis 180 °C, vorzugsweise 50 bis 150 °C, vorerhitzt. Wesentlich ist einzig und allein, daß in dem Mischgefäß und der gegebenenfalls nachgeschalteten Reaktionszone eine Temperatur von 120 bis 300 °C, vorzugsweise 150 bis 250 °C, herrscht. Unter diesen Temperaturbedingungen erfolgt bei der Durchführung des erfindungsgemäßen Verfahrens spontan eine einstufige Phosgenierung des eingedüsten Amins, so daß im Extremfall das Mischgefäß auch ohne nachgeschaltete Reaktionszone die Doppelfunktion eines Misch- und Reaktionsgefäßes übernehmen kann. Falls auf die Nachschaltung einer weiteren Reaktionszone verzichtet werden soll, ist es allerdings zweckmäßig, die Dimension des Mischgefäßes so zu bemessen, daß der Abstand vom Düsenaustritt bis zur, der Düse gegenüberliegenden, Begrenzung des Mischgefäßes mindestens das 200-fache des Düsendurchmessers beträgt.

Oft ist es jedoch zweckmäßig, dem Mischgefäß eine weitere Reaktionszone nachzuschalten. Eine derartige Reaktionszone kann aus röhrenförmig gestalteten Reaktoren, Rohrbündelreaktoren oder anderen üblichen Apparaturen, wie beispielsweise Kesselkaskaden, bestehen. Diese gegebenenfalls mit dem Ausgang des Mischgefäßes verbundene Reaktionszone kann, wie dies in Fig. 2 beispielhaft angedeutet ist, mit einer Ummantelung versehen sein, die eine Beheizung der Reaktionszone gestattet. Selbstverständlich kann die Reaktionszone auch auf andere Weise beheizbar sein.

Bei der Durchführung des erfindungsgemäßen Verfahrens ist die Phosgenierungsreaktion im allgemeinen nach einer mittleren Verweilzeit von 5 Sekunden bis 5 Minuten im Mischgefäß und der gegebenenfalls nachgeschalteten Reaktionszone beendet. Zur Aufarbeitung des Reaktionsgemisches wird das das Mischgefäß bzw. die nachgeschaltete Reaktionszone verlassende Reaktionsgemisch über das Auslaßventil im allgemeinen einstufig entspannt, wobei gleichzeitig ein durch die Druckentspannung hervorgerufener Temperaturabfall um ca. 50 bis 150 °C eintritt. Die Entspannung auf Normaldruck kann aber auch stufenweise in zwei oder mehreren Stufen erfolgen, um die Gasmengen in jeder Stufe möglichst klein zu halten. Das das Auslaßventil verlassende Gemisch wird in einem Entspannungsgefäß in eine gasförmige und eine flüssige Phase aufgetrennt. Bei der erfindungsgemäß bevorzugten Herstellung von organischen Mono- oder Polyisocyanaten, die unter Normaldruck einen Siedepunkt von mindestens 100 °C aufweisen, besteht die hierbei anfallende Gasphase in erster Linie aus überschüssigem Phosgen und während der Phosgenierungsreaktion gebildetem Chlorwasserstoff. Geringe Mengen an mitgerissenem Lösungsmittel und Isocyanat werden der Gasphase durch fraktionierte Kondensation in einem geeigneten Kühler entnommen. Das Kondensat kann mit der im Entspannungsgefäß anfallenden flüssigen Phase vereinigt werden. Die flüssige Phase wird in an sich bekannter Weise destillativ unter Reindarstellung des Mono- oder Polyisocyanats aufgearbeitet. Selbstverständlich stellt die beschriebene Art der Aufarbeitung der beim erfindungsgemäßen Verfahren anfallenden Reaktionsgemische nur eine unter mehreren im Prinzip denkbaren Varianten dar. Die Art der Aufarbeitung der beim erfindungsgemäßen Verfahren anfallenden Reaktionsgemische ist in keiner Weise kritisch und kann nach den üblichen Methoden des Standes der Technik erfolgen.

Die nachfolgenden erfindungsgemäßen Ausführungsbeispiele werden in einer Vorrichtung gemäß Fig. 2 durchgeführt. Dies bedeutet, daß die Mischkammer (15) mit einem nachgeschalteten Rohrreaktor kombiniert zum Einsatz gelangt. Alle Prozentangaben beziehen sich auf Gewichtsprozente.

Beispiel 1

In einem mit einem Rohrreaktor kombinierten, rohrförmig gestalteten Mischgefäß (15) mit einem inneren Durchmesser von 25 mm und einem Gesamtvolumen des Mischgefäßes und des Rohrreaktors von 7,3 l werden kontinuierlich 36,6 lg/h einer 15 %igen 1,6-Diaminohexan-Lösung in o-Dichlorbenzol und 120 kg/h einer 44,6 %igen Phosgenlösung in o-Dichlorbenzol zur Reaktion gebracht. Die Vermischung der auf 180 °C erhitzten Phosgenlösung mit der auf 130 °C erhitzten Aminlösung erfolgt durch Eindüsen der Aminlösung mittels einer Glattstrahldüse (2), die einen lichten Durchmesser von 0,5 mm und eine Länge von 3 mm aufweist, in das Mischgefäß, welches von der kontinuierlich eingespeisten Phosgenlösung blasenfrei ausgefüllt ist. Der Abstand von der Düse bis zu gegenüberliegenden, mit dem Rohrreaktor verbundenen Ausgangsöffnung des Mischgefäßes beträgt 30 cm. Der Abstand des Düsenstrahls zur Seitenwand des Mischgefäßes beträgt 12,25 mm. Das Mischgefäß und der Rohrreaktor, sowie die Phosgenlösung, stehen unter einem Druck von 60 bar ; die Aminlösung steht am Eingang zur Düse unter einem Druck von 81 bar. Über das Auslaßventil (17) wird dem Rohrreaktor kontinuierlich Reaktionsgemisch entnommen und in dem Abscheider (19) in Gasphase (21) und Flüssigphase (18) aufgetrennt. Mitgerissene Anteile an flüssigen Bestandteilen werden der Gasphase im Kühler (20) durch fraktionierte Kondensation entzogen und in die Flüssigphase zurückgeführt.
Durch destillative Aufarbeitung der kombinierten Flüssigphasen erhält man 7,83 kg/h 1,6-Diisocyanatohexan, entsprechend einer Ausbeute von 98,5 %, bezogen auf eingesetztes Diamin.

Beispiel 2

Beispiel 1 wird wiederholt, jedoch unter Aufrechterhaltung eines Drucks in der Misch- und Reaktionszone von 30 bar und einem Druck der Aminlösung vor der Glattstrahldüse von 58 bar. Die Ausbeute an 1,6-Diisocyanatohexan beträgt 7,7 kg/h, entsprechend 97 % der Theorie.

Beispiel 3

Es wird eine der in Beispiel 1 verwendeten Apparatur analoge Vorrichtung verwendet, jedoch wird das Volumen des Röhrenreaktors so bemessen, daß das Gesamtvolumen des Mischraums und des Röhrenreaktors lediglich 0,9 l beträgt. Der Düsendurchmesser liegt ebenfalls bei 0,5 mm.
Wie in Beispiel 1 beschrieben werden 40 kg/h einer auf 140 °C vorerhitzte, 17,8 %ige 1,6-Diaminohexan-Lösung in o-Dichlorbenzol mit 120 kg/h einer auf 210 °C vorerhitzten 61,7 %igen Phosgenlösung in o-Dichlorbenzol bei einem Reaktordruck von 60 bar zur Reaktion gebracht. Die Aminlösung wird hierbei vor Eintritt in die Düse auf einem Druck von 80 bar gehalten. Die Reaktionstemperatur im Mischraum und in der nachgeschalteten Reaktionszone wird auf 220 °C gehalten. Pro Stunde fallen 9,91 kg 1,6-Diisocyanatohexan an. Dies entspricht 96 % der Theorie, bezogen auf eingesetztes Diamin.

Beispiel 4

Wie in Beispiel 3 beschrieben werden 54 kg/h einer auf 100 °C vorerhitzten 16,8 %igen 1,6-Diaminohexan-Lösung in o-Dichlorbenzol mit 108 kg/h einer auf 200 °C vorerhitzten 43,9 %igen Phosgenlösung zur Reaktion gebracht. Der Düsendurchmesser beträgt 0,5 mm. Der Reaktordruck liegt bei 60 bar, der Druck der Aminlösung vor Eintritt in die Düse bei 88 bar. Die Temperatur in der Misch- und Reaktionszone liegt bei 221 °C. Es fallen 12,3 kg/h 1,6-Diisocyanatohexan, entsprechend 93,8 % der Theorie, an.

Beispiel 5

In diesem Beispiel wird eine den vorhergehenden Beispielen analoge Apparatur verwendet, wobei jedoch das Volumen des dem Mischgefäß nachgeschalteten Röhrenreaktors so bemessen wird, daß das Gesamtvolumen aus Mischgefäß und Röhrenreaktor 1,55 l beträgt. Die Düse weist einen Durchmesser von 0,4 mm auf.
33 kg/h einer auf 135 °C vorerhitzten, 22 %igen 1,6-Diaminohexan-Lösung in o-Dichlorbenzol werden mit 123 kg/h einer auf 190 °C vorerhitzten 62,2 %igen Phosgenlösung in o-Dichlorbenzol umgesetzt. Der Druck in der Misch- und Reaktionszone liegt bei 50 bar ; der Druck der Aminlösung vor Eintritt in die Düse liegt bei 75 bar. Die Temperatur in der Misch- und Reaktionszone liegt bei ca. 200 °C. Es fallen 9,89 kg/h 1,6-Diisocyanatohexan, entsprechend 94,9 % der Theorie, an.

Beispiel 6

Es wird die in Beispiel 1 beschriebene Apparatur mit den dort genannten Abmessungen verwendet, wobei jedoch die Düse einen Durchmesser von 0,55 mm aufweist.
53,6 kg/h einer auf 160 °C vorerhitzten, 18 %igen Lösung von 4,4'-Diaminodiphenylmethan in Chlorbenzol werden mit 96,4 kg/h einer auf 190 °C vorerhitzten Phosgenlösung in Chlorbenzol zur Reaktion gebracht. Der Druck in der Misch- und Reaktionszone liegt bei 65 bar. Der Druck der Aminlösung vor Eintritt in die Düse liegt bei 95 bar. Es fallen 11,7 kg/h 4,4'-Diisocyanatodiphenylmethan, entsprechend einer Ausbeute von 96 % der Theorie, an.

Beispiel 7

Es wird eine den bisherigen Vorrichtungen entsprechende Vorrichtung verwendet, wobei jedoch das Volumen des Röhrenreaktors so bemessen ist, daß das Gesamtvolumen aus Mischraum und nachgeschalteter Reaktionszone 3,7 l beträgt. Der Düsendurchmesser liegt bei 0,4 mm.

42,2 kg/h einer auf 172 °C vorerhitzten, 46,7 %igen Lösung eines Gemisches aus 2,4-Diaminotoluol und 2,6-Diaminotoluol im Gewichtsverhältnis 1,86 : 1 in o-Dichlorbenzol werden mit 123,6 kg/h einer auf 216 °C vorerhitzten 80 %igen Lösung von Phosgen in o-Dichlorbenzol zur Reaktion gebracht. Der Druck in der Misch- und Reaktionszone liegt bei 60 bar. Der Druck der Aminlösung vor Eintritt in die Düse liegt bei 110 bar.

Die Temperatur in der Misch- und Reaktionszone liegt bei 198 °C. Es werden 16,8 kg/h Diisocyanatotoluol, entsprechend einer Ausbeute von 97 % der Theorie, erhalten.

**Ansprüche**

1. Verfahren zur kontinuierlichen Herstellung von organischen Mono- oder Polyisocyanaten in einstufiger Reaktion durch kontinuierliche Vereinigung von Lösungen primärer Mono- oder Polyamine in inerten organischen Lösungsmitteln mit überschüssigen Mengen an, in einem inerten organischen Lösungsmittel gelöstem, Phosgen bei erhöhtem Druck und erhöhter Temperatur in einem Mischraum (4) und kontinuierliche Aufarbeitung der den Mischraum kontinuierlich verlassenden vereinigten Lösungen, gegebenenfalls nachdem diese eine dem Mischraum nachgeschaltete Reaktionszone durchlaufen haben, dadurch gekennzeichnet, daß man die im Überschuß zum Einsatz gelangende Phosgenkomponente kontinuierlich in dem Mischraum vorlegt und die im Unterschuß zum Einsatz gelangende Aminkomponente mittels mindestens einer Glattstrahldüse (2) mit einem lichten Durchmesser von 0,1 bis 30 mm unter Einhaltung eines Differenzdrucks von mindestens 0,5 bar in die vorgelegte Phosgenlösung eindüst.

2. Verfahren gemäß Anspruch 1, dadurch gekennzeichnet, daß man die Vereinigung der Lösungen und die im Mischraum und der gegebenenfalls im Mischraum nachgeschalteten Reaktionszone ablaufende Umsetzung unter einem Druck von 10 bis 1 000 bar durchführt.

3. Verfahren gemäß Ansprüchen 1 und 2, dadurch gekennzeichnet, daß man die Vereinigung der Lösungen und die Umsetzung unter einem Druck von 25 bis 150 bar durchführt.

4. Verfahren gemäß Ansprüchen 1 bis 3, dadurch gekennzeichnet, daß man die Vereinigung der Lösungen und die im Mischraum und gegebenenfalls der dem Mischraum nachgeschalteten Reaktionszone ablaufende Phosgenierungsreaktion bei 120 bis 300 °C durchführt.

5. Verfahren gemäß Ansprüchen 1 bis 4, dadurch gekennzeichnet, daß man die Vereinigung der Lösungen und die Reaktion bei einer Temperatur von 150 bis 250 °C durchführt.

6. Verfahren gemäß Ansprüchen 1 bis 5, dadurch gekennzeichnet, daß man die Reaktionspartner in einem Molverhältnis Phosgen : primäre Aminogruppen von 2 : 1 bis 30 : 1 entsprechenden Mengen einsetzt.

**Claims**

1. Process for the continuous production of organic mono- or poly-isocyanates in a single-stage reaction by continuously combining solutions of primary mono- or poly-amines in inert organic solvents with excess quantities of phosgene dissolved in an inert organic solvent, under an elevated pressure and at an elevated temperature in a mixing chamber (4) and continuously working up the combined solutions continuously leaving the mixing chamber, optionally after they have passed through a reaction zone following the mixing chamber, characterised in that the phosgene component used in excess is continuously introduced into the mixing chamber and the amine component used in a sub-stoichiometric quantity is sprayed into the phosgene solution by means of at least one smooth-jet nozzle (2) having an internal diameter of 0.1 to 30 mm, a differential pressure of at least 0.5 bar being maintained.

2. Process according to Claim 1, characterised in that the combination of the solutions and the reaction taking place in the mixing chamber and the reaction zone optionally following the mixing chamber are carried out under a pressure of 10 to 1,000 bars.

3. Process according to Claims 1 and 2, characterised in that the combination of the solutions and the reaction are carried out under a pressure of 25 to 150 bars.

4. Process according to Claims 1 to 3, characterised in that the combination of the solutions and the phosgenation reaction taking place in the mixing chamber and the reaction zone optionally following the mixing chamber are carried out at 120 to 300 °C.

5. Process according to Claims 1 to 4, characterised in that the combination of the solutions and the reaction are carried out at a temperature of 150 to 250 °C.

6. Process according to Claims 1 to 5, characterised in that the reactants are used in quantities corresponding to a molar ratio of 2 : 1 to 30 : 1 of phosgene to primary amino groups.

**Revendications**

1. Procédé de préparation en continu de mono-isocyanates ou de polyisocyanates organiques dans une réaction à une seule étape par la réunion continue de solutions de mono-amines ou de polyamines primaires dans des solvants organiques inertes avec des quantités excessives de phosgène dissous dans un solvant organique inerte, sous forte pression et à température élevée dans un compartiment de mélange (4) et en traitant continuellement les solutions combinées quittant continuellement le compartiment de mélange, éventuellement après que ces solutions aient traversé une zone réactionnelle située en aval du compartiment de mélange, caractérisé en ce qu'on dépose continuellement, dans le compartiment de mélange, le composant phosgène devant être utilisé en excès tandis que, dans la solution de phosgène préalablement déposée, on injecte le composant aminé utilisé en déficit au moyen d'au moins une buse à jet lisse (2) ayant un diamètre intérieur de 0,1 à 30 mm, tout en maintenant une pression différentielle d'au moins 0,5 bar.

2. Procédé suivant la revendication 1, caractérisé en ce qu'on effectue la combinaison des solutions et la réaction se déroulant dans le compartiment de mélange et dans la zone réactionnelle éventuellement prévue en aval de ce compartiment de mélange, sous une pression de 10 à 1 000 bars.

3. Procédé suivant les revendications 1 et 2, caractérisé en ce qu'on effectue la combinaison des solutions et la réaction sous une pression de 25 à 150 bars.

4. Procédé suivant les revendications 1 à 3, caractérisé en ce qu'on effectue la combinaison des solutions et la réaction de phosgénation se déroulant dans le compartiment de mélange et la zone réactionnelle éventuellement prévue en aval de ce compartiment de mélange, à une température de 120 à 300 °C.

5. Procédé suivant les revendications 1 à 4, caractérisé en ce qu'on effectue la combinaison des solutions et la réaction à une température de 150 à 250 °C.

6. Procédé suivant les revendications 1 à 5, caractérisé en ce qu'on utilise les partenaires réactionnels en quantités correspondant à un rapport molaire phosgène/groupes amino primaires de 2 : 1 à 30 : 1.

FIG.1

FIG. 2